## Europäisches Patentamt

## European Patent Office

## Office européen des brevets

(19)

(11) Veröffentlichungsnummer : **0 136 470**
**B1**

(12)

# EUROPÄISCHE PATENTSCHRIFT

(45) Veröffentlichungstag der Patentschrift :
21.12.88

(21) Anmeldenummer : 84109243.0

(22) Anmeldetag : 03.08.84

(51) Int. Cl.⁴ : **A 61 K 31/19**, C 07 C 59/84,
C 07 C 59/64,
C 07 C101/453, C 07 C101/24

(54) Injizierbare Lösung zur Behandlung von Entzündungen.

(30) Priorität : 05.08.83 DE 3328401

(43) Veröffentlichungstag der Anmeldung :
10.04.85 Patentblatt 85/15

(45) Bekanntmachung des Hinweises auf die Patenterteilung : 21.12.88 Patentblatt 88/51

(84) Benannte Vertragsstaaten :
AT BE CH DE FR GB IT LI LU NL SE

(56) Entgegenhaltungen :
BE—A— 882 889
DE—A— 2 508 895
US—A— 3 904 682
US—A— 4 279 926
CHEMICAL ABSTRACTS, Band 94, no. 8, Februar 23,
1981, no. 52802y, COLUMBUS OHIO (US), J. IBANEZ
et al.: "Lysinate of naproxen: pharmacokinetic profile
and pharmacological comparison"
J. Ibanez et al., Arch. Farmacol. Toxikol. 1980, 6(2),
145-150

(73) Patentinhaber : **Merckle GmbH**
**Dr.-Georg-Spohn-Strasse 7**
**D-7902 Blaubeuren (DE)**

(72) Erfinder : **Metz, Gunter, Dr.**
**Auf dem Rucken 29**
**D-7902 Blaubeuren (DE)**

(74) Vertreter : **Lederer, Franz, Dr. et al**
**Van der Werth, Lederer & Riederer Patentanwälte**
**Lucile-Grahn-Strasse 22**
**D-8000 München 80 (DE)**

**0 136 470**

**Beschreibung**

Injektionslösungen zur Behandlung akuter Entzündungen und rheumatischer Erkrankungen sind überwiegend Kombinationspräparate basierend auf entzündungshemmenden Wirkstoffen, deren Einarbeitung in physiologisch verträgliche Lösungen problemlos ist, wie z. B. Phenylbutazon und Salicylsäurederivate. Daneben sind insbesondere auch Kombinationen mit Corticoiden üblich, deren Einsatz jedoch therapeutisch Grenzen gesetzt sind.

Von den anerkannten potenten antirheumatisch wirksamen Monosubstanzen, wie Indometacin, Ketoprofen, Naproxen, Ibuprofen und Diclofenac ist in einer gebrauchsfertigen Injektionslösung nur das Diclofenac-Natrium bekannt. Indometacin und Ketoprofen stehen zur therapeutischen Anwendung derzeit nur als Trockenlyophilisate zur Verfügung. Dies hat einerseits seine Ursache darin, daß zur Solubilisierung mit anorganischen Basen, z. B. Natriumhydroxid, relativ hohe pH-Werte erforderlich sind, andererseits bei diesen pH-Werten die Wirkstoffe zersetzt werden und instabile Formulierungen ergeben.

Erfindungsgemäße Aufgabe war daher die Herstellung einer stabilen und physiologisch verträglichen gebrauchsfertigen Injektionslösung mit möglichst hohem Wirkstoffanteil in kleinen Volumina. Bei der Auswahl geeigneter Wirkstoffe wurde berücksichtigt daß Indometacin zwar sehr potent, aber mit hohen Nebenwirkungsraten belastet ist und Ibuprofen als wirkungsmäßig schwächste Substanz der genannten Wirkstoffe gilt und zur Erzielung therapeutischer Effekte sehr hohe Konzentrationen in der Injektionslösung und damit große Volumina erfordert hätte. Die erfindungsgemäße Aufgabe konnte durch Einsatz des L-Lysinats von Diclofenac gelöst werden. Die einfach herzustellende Lösung des Lysinats hat gegenüber der entsprechenden Lösung des Natriumsalzes den Vorteil, daß ihr pH-Wert im physiologischen Bereich liegt, daß sie deutlich höhere Wirkstoffkonzentrationen erlaubt und daß die so erzielten Lösungen stabil sind. Wie weiterhin überraschend in experimentellen Untersuchungen gefunden wurde, ist die Lösung des Lysinats der des entsprechenden Natriumsalzes in der pharmakologischen Wirkung signifikant überlegen.

Diclofenac ist der international eingeführte Trivialname folgendes Wirkstoffs :

Diclofenac : [2-(2,6-Dichlor-anilino)-phenyl] essigsäure.

Lysin ist die Aminosäure mit der chemischen Bezeichnung 2,6-Diaminocapronsäure, die erfindungsgemäß in der natürlich vorkommenden L-Form eingesetzt wird. Die Lysinate sind die Salze des Lysins.

Die Wirksamkeitsbestimmung der erfindungsgemäßen Lösung im Vergleich zu den entsprechenden Lösungen der Natriumsalze erfolgte nach einer modifizierten Methode von Coubon et al., 1954 (R.A. Turner Screening Methods in Pharmacology, Academic Press New York, 1965, S. 157) am Kaolinödem der Rattenpfote (7 Tiere/Gruppe). Der Provokator Kaolin wurde als 20 %ige wäßrige Suspension in einem Volumen von 0,2 ml/Pfote subcutan in die rechte Hinterpfote appliziert. 1 Stunde nach der Provokatorgabe erfolgte die i.m.-Applikation der Testlösungen in den rechten Oberschenkel. Die Kontrollgruppe erhielt jeweils das Vehikel. 24 Stunden nach Applikation wurde die Ödembildung durch Messung der Wasserverdrängung nach bekannten Verfahren gemessen. Hierbei wurden die in folgender Tabelle zusammengefaßte Resultate erzielt :

| Injektionslösung | | Wirkstoffdosis mg/kg | Ödemreduktion[1] |
|---|---|---|---|
| Diclofenac | -Lysinat | 0,75 | Diclofenac-Natrium - 1,7 % |
| | | 7,5 | Diclofenac-Natrium - 44,8 % *** |
| | -Natrium | 10 | Diclofenac-Natrium - 10,3 % |
| | | 20 | Diclofenac-Natrium - 39,7 % *** |

Signifikanz*** p < 0,001
1) bezogen auf Kontrollgruppe (100 %)

Wie die Ergebnisse zeigen, ist das verwendete Lysinat dem Natriumsalz in wesentlich geringerer Wirkstoffkonzentration entzündungshemmend signifikant überlegen.

Aus der DE-A 2 508 895 und der US-P 4 279 926 sind Ketoprofenlysinat und Naproxenlysinat bekannt, wobei die racemische DL-Form ausdrücklich gegenüber der natürlichen L-Form bevorzugt wird.

Auch in Chemical Abstracts 94, 52802 y wird Naproxenlysinat als entzündungshemmender und analgetischer Wirkstoff genannt.

Naproxensalze darunter auch das Lysinat werden in der US-P 3 904 682 offenbart.

Ketoprofenlysinat wird auch in der BE-P 882 889 beschrieben.

Die Herstellung der erfindungsgemäßen Injektionspräparate erfolgt vorzugsweise durch Lösung des isolierten Lysinats. Die Bildung des Lysinats ist durch Reaktion des Natriumsalzes von Diclofenac mit

2

Lysin Hydrochlorid in geeigneten Lösungsmitteln nach üblichen Verfahren möglich. Das kristalline Lysinat kann ferner durch Kristallisation aus geeigneten Lösungsmitteln rein dargestellt werden.

Ferner kann die Umsetzung des gelösten Natriumsalzes mit äquimolaren Mengen an Lysin Hydrochlorid ohne vorherige Isolation der gebildeten Lysinate unmittelbar in der fertigen Injektionslösung erfolgen, sofern das dabei gebildete Natriumchlorid physiologisch verträgliche Werte nicht überschreitet.

Die Umsetzung und Solubilisierung des Wirkstoffs mit Lysin Base direkt in der Injektionslösung ist ebenfalls möglich, aber wenig vorteilhaft. Da Lysin Base selbst zur Eigenverfärbung und Zersetzung neigt, ist der Einsatz des stabilen Lysin Hydrochlorids dem der Base vorzuziehen.

Als Lösungsmittel dient Wasser ad inj. alleine oder bevorzugt unter Zusatz üblicher und physiologisch verträglicher Lösungsmittel und/oder Lösungsvermittler wie z. B. Propylenglykol, Polyole wie Glycerin, Polyoxyalkylenen z. B. Poly(oxethylen)-poly(oxpropylen) polymeren, Glyzerinformal, Benzylalkohol oder Butandiol. Durch Zusatz dieser Lösungsvermittler werden bekannterweise Lösungen erzielt, die trotz hoher Wirkstoffkonzentration in der Kälte stabil sind und nicht zu teilweiser Kristallisation der Wirkstoffe führen.

Weiterhin können, wie für Injektionspräparate üblich, Oxidationsstabilisatoren, wie z. B. Natriumdisulfit, oder Konservierungsmittel, wie z. B. Benzylalkohol, sinnvoll eingearbeitet werden. Für die therapeutische Anwendung ist ferner die Zugabe von Lokalanästhetika, wie Lidocain oder Cinchocain, empfehlenswert.

Die erfindungsgemäßen Injektionspräparate werden für die therapeutische Anwendung nach üblichen Verfahren sterilisiert oder keimfrei in Ampullen von 1-3 ml abgefüllt.

Die erfindungsgemäßen Injektionslösungen enthalten 2-5 % an Diclofenac. Die therapeutisch verwendete Einzeldosierung beträgt 40-100 mg an Diclofenac.

Beispiel

3,75 g Diclofenac Natrium
2,15 g L-Lysin Hydrochlorid
60,0 ml Propylenglykol
1,0  g Benzylalkohol
ad 100  ml Aqua bidest.

**Patentansprüche**

1. Injizierbare Lösung enthaltend den entzündungshemmenden Wirkstoff Diclofenac in Form eines L-Lysinats.

2. Injektionslösung gemäß Anspruch 1, enthaltend Diclofenac-L-Lysinat in einer Menge von 2-5 Gew.-% bezogen auf Diclofenac.

3. Injektionslösung nach einem der Ansprüche 1 oder 2 als wäßrige Lösung oder als wäßriges Lösungsmittelgemisch unter Zusatz physiologisch verträglicher Lösungsvermittler, Stabilisatoren und/oder Konservierungsmittel.

4. Injektionslösung nach Anspruch 3 zusätzlich enthaltend ein geeignetes Lokalanästhetikum.

5. Verwendung von Diclofenac-L-Lysinat zur Herstellung injizierbarer Lösungen.

6. Verwendung von Diclofenac-L-Lysinat zur Herstellung einer injizierbaren Lösung zur Behandlung von Entzündungen und rheumatischen Erkrankungen.

**Claims**

1. Injectable solution containing the inflammation-inhibiting active ingredient diclofenac in the form of an L-lysinate.

2. Injection solution according to claim 1, containing diclofenac-L-lysinate in an amount of 2-5 weight % based on diclofenac.

3. Injection solution according to one of claims 1 or 2 as an aqueous solution or as an aqueous solvent mixture with addition of physiologically acceptable solubilising agents, stabilisers and/or preserving agents.

4. Injection solution according to claim 3 additionally containing a suitable local anaesthetic.

5. Use of diclofenac-L-lysinate for the preparation of injectable solutions.

6. Use of diclofenac-L-lysinate for the preparation of an injectable solution for the treatment of inflammation and rheumatic diseases.

**Revendications**

1. Solution pour injection contenant le principe actif anti-inflammatoire diclofénac sous la forme d'un L-lysinate.

2. Solution pour injection selon la revendication 1, contenant du L-lysinate de diclofénac en une quantité de 2-5 % en poids rapportée au diclofénac.

3. Solution pour injection selon l'une des revendications 1 ou 2, sous la forme d'une solution aqueuse ou d'un mélange aqueux de solvants, avec addition de tiers-solvants, de stabilisants et/ou d'agents conservateurs physiologiquement tolérés.

4. Solution pour injection selon la revendication 3, contenant en outre un anesthésique local approprié.

5. Utilisation du L-lysinate de diclofénac pour préparer des solutions injectables.

6. Utilisation du L-lysinate de diclofénac pour préparer une solution injectable destinée au traitement des inflammations et des maladies rhumatismales.